# EUROPEAN PATENT APPLICATION

(11) **EP 2 878 326 A1**
(43) Date of publication of application: **03.06.2015**
(21) Application number: 13194695.6
(22) Date of filing: 27.11.2013
(51) Int. Cl.: A61M 15/00

(54) **Inhaler**

(71) Applicant: Vectura Delivery Devices Limited, Chippenham, Wiltshire SN14 6FH (GB)
(72) Inventor: Deamer, John David, Chippenham, Wiltshire SN14 6FH (GB); McGuiness, Liam Phillip, Chippenham, Wiltshire SN14 6FH (GB)
(74) Representative: Clarke, Christopher John

(57) **Abstract**

An inhaler is provided which comprises a housing to receive a strip comprising a plurality of blisters having pierceable lids and containing medicament for inhalation; a mouthpiece mounted to the housing through which the medicament is inhaled by a user; a piercing element; an actuator for moving the blisters sequentially into alignment with the piercing element and for subsequently causing the piercing element to pierce the lid of the aligned blister so that when the user inhales through the mouthpiece, an airflow is generated through the blister to entrain the medicament and carry it, via the mouthpiece, into the user's airway; and a mechanism for applying a biasing force to the actuator; wherein the mechanism is adapted to apply a biasing force to the actuator during a forward stroke which is independent of the biasing force that it applies to the actuator during a return stroke.

## Description

### Technical Field of the Invention

The present invention relates to an inhaler for oral or nasal delivery of powdered medicament from a pierceable container, such as a blister. In particular, the invention relates to an inhaler having an actuating mechanism which comprises a mechanism for controlling the force that the user applies during actuation.

### Background to the Invention

Inhalers provide an attractive method for administering drugs (for example to treat local diseases of the airway or to deliver drugs to the bloodstream via the lungs) because these devices are relatively easy for a patient to use discreetly and in public. The medicament is commonly provided as a dry powder pre-packaged in individual doses, usually in the form of capsules or blisters. It is advantageous for the inhaler to be capable of holding a number of doses so that there is no need to insert a blister into the device each time it is used. Therefore, many inhalers include means for storing a number of doses, e.g. in the form of a blister strip.

WO12/069854 discloses an inhaler for use with a blister strip. The user applies pressure to an actuating lever so that it rotates through a first portion of its stroke to an intermediate position, thereby moving an unused blister into alignment with a piercing element. The actuating lever is then rotated through a second portion of its stroke beyond the intermediate position, during which the blister strip remains stationary but the mouthpiece pivots, so that the piercing element pierces the lid of the aligned blister. The user then inhales through the mouthpiece, and finally returns the actuating lever to its initial position. The blister strip remains stationary during this return movement. The inhaler includes a detent mechanism for holding the actuating lever at the end of its forward stroke. The detent mechanism resists the motion of the actuating lever near the end of the second portion of the forward stroke. A small force must be applied to the actuating lever, firstly to overcome the resistance caused by the detent mechanism at the end of the forward stroke and secondly to overcome the hold placed on the actuating lever by the detent mechanism at the start of the return stroke. In addition to providing some resistance to the movement of the actuating lever, the detent mechanism also generates an audible click at the end of the forward stroke and so provides a clear signal to the user that the end of travel of the actuating lever has been reached.

### Brief Description of the Invention

At any particular point, the detent mechanism of WO12/069854 applies the same biasing force to the actuating lever regardless of whether the actuating lever is on the forward or return stroke. However, the present inventors have now recognized that it would be advantageous for the force to be different for the forward and return strokes. The present invention solves this problem by providing a mechanism which can apply different biasing forces on the forward and return strokes.

Accordingly the present invention provides an inhaler comprising:
- a housing to receive a strip comprising a plurality of blisters having pierceable lids and containing medicament for inhalation;
- a mouthpiece mounted to the housing through which the medicament is inhaled by a user;
- a piercing element;
- an actuator for moving the blisters sequentially into alignment with the piercing element and for subsequently causing the piercing element to pierce the lid of the aligned blister so that when the user inhales through the mouthpiece, an airflow is generated through the blister to entrain the medicament and carry it, via the mouthpiece, into the user's airway; and
- a mechanism for applying a biasing force to the actuator;
characterised in that the mechanism is adapted to apply a biasing force to the actuator during a forward stroke which is independent of the biasing force that it applies to the actuator during a return stroke.

Preferably the mechanism for applying the biasing force comprises a cam follower on the actuator and a track on the housing, wherein the track comprises first and second track portions which are separated by a dividing wall, wherein the two track portions are differently shaped. The cam follower contacts and moves along the first track portion on the forward stroke of the actuator and then moves along the second track portion on the return stroke. The track portions are shaped to change the degree of deflection and / or retraction of the cam follower during the forward and / or return strokes of the actuator. The deflection and / or retraction of the cam follower generates the biasing force applied to the actuator. This biasing force must be overcome by the user to cause the actuator to move and hence to operate the inhaler.

Preferably, the cam follower is deflected or retracted by contact with the track portion as a result of the cam follower being inherently resilient. Alternatively or additionally, the cam follower may be resiliently mounted on the actuating lever.

Preferably each end of the dividing wall has a ramp to guide the cam follower at the end of each stroke of the actuator from one track portion onto the other track portion for the next stroke.

Preferably the cam follower has a body part and a head. More preferably the head of the cam follower is rounded.

Preferably the body part of the cam follower comprises a region of reduced thickness, more preferably a line of reduced thickness across the body part, so that when the head contacts the dividing wall, the cam follower bends out of the plane of the dividing wall along this region.

Optionally, the first track portion is shaped such that the biasing force applied to the actuator during the forward stroke gradually increases until the point at which piercing element begins to pierce the lid of the blister. Preferably the first track portion is shaped so that the biasing force applied to the actuator is gradually released thereafter.

Preferably the first and / or second track portions are shaped so that the biasing force urges the actuator into a fully actuated and / or fully returned position respectively. In particular this can be achieved by means of a hump or protrusion situated near to the end of the respective track portion.

Preferably the cam follower is in contact with the first and / or second track portions, so that a biasing force is applied to the actuator, throughout a substantial portion of the forward and / or the return strokes of the actuator.

Preferably the piercing element is mounted for rotation about a first axis and the actuator is an actuating lever mounted for rotation about a second axis, wherein the actuating lever cooperates with the piercing element so that the piercing element pivots about the first axis to pierce the lid of the aligned blister in response to rotation of the actuating lever about the second axis from its initial position.

### Detailed Description of the Invention

Embodiments of the invention will now be described, by way of example only, with reference to Figures 1 to 4 of the accompanying drawings, in which:
FIGURES 1A and 1B show an inhaler according to the invention;
FIGURES 2A and 2B show a track which has first and second portions separated by a dividing wall;
FIGURES 3A and 3B are schematic side views of the first and second track portions;
FIGURE 4 shows the cam follower together with the track.

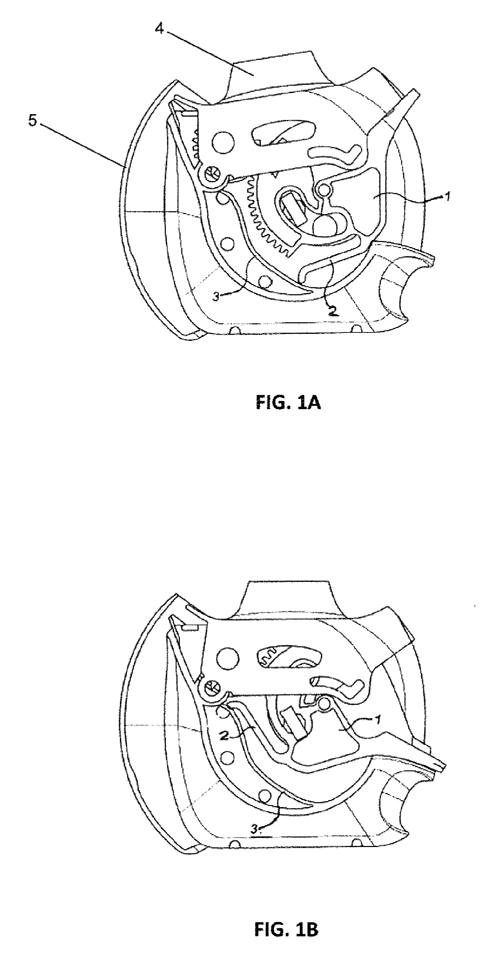

Figure 1 shows an inhaler having a housing which contains a blister strip (not shown). The blister strip is typically cold formed from a ductile foil laminate or a plastics material and includes a pierceable lid which is heat-sealed around the periphery of the blister after the dose of medicament has been introduced during manufacture. The inhaler has an actuator 1 which is an actuating lever and which includes a cam follower 2. The inhaler also has a track 3, a mouthpiece 4 and a cap 5.

The mouthpiece is mounted to the outside of the housing. Although the term "mouthpiece" is used, the invention is also applicable to devices in which the dose is inhaled through the nasal passages. Therefore "mouthpiece" should also be construed so as to include within its scope a tube which is inserted into the nasal passages of a patient for inhalation therethrough.

The mouthpiece has a piercing element (not shown) on its lower side i.e. facing the blister strip. The piercing element may be fixed to the mouthpiece which is pivotally mounted to the housing so that the mouthpiece pivots, together with the piercing element, about a first axis in response to operation of the actuating lever. Alternatively, the piercing element may be pivotally mounted to the mouthpiece so that it pivots about the first axis relative to the mouthpiece in response to operation of the actuating lever.

The piercing element is designed to create multiple openings in the lid of the blister so that air can be drawn into the blister through one or some of the openings and flow out of the blister together with an entrained dose of medicament, through one or more other openings and via the mouthpiece into the user's airway. The piercing element may comprise a number of blades, spikes or the like.

The actuating lever is mounted for rotation on the housing and is pivotable about a second axis. The actuating lever may comprise a protruding button to aid actuation of the lever by the user. Operation of the actuating lever sequentially moves each blister into alignment with the piercing element and causes the piercing element to pierce the lid of the aligned blister.

Preferably the actuating mechanism is configured such that rotation of the actuating lever through a first portion of its forward stroke moves the blister into alignment with the piercing element and such that further rotation of the actuating lever in the same direction, during a second portion of its forward stroke, causes the piercing element to pierce the lid of the aligned blister. As described in WO12/069854, this may be achieved by means of a cam drive member located on one of the actuating lever and mouthpiece which cooperates with a cam surface on the other. The cam surface has an arcuate portion having an axis that corresponds to the second axis about which the actuating lever rotates, followed by a leg portion. During rotation of the actuating lever through the first portion of its forward stroke, the cam drive member slides along the arcuate portion of the cam surface without causing any movement of the mouthpiece because the arcuate portion has the second axis as its radius. However, during subsequent rotation of the actuating lever, the cam drive member reaches the leg portion and engages the side walls of the cam surface so as to cause the mouthpiece to rotate about the first axis together with the actuating lever, thereby pulling the piercing element into the aligned blister and piercing it.

The inhaler preferably has a blister strip drive wheel and indexing mechanism as described in WO09/092652. The blister strip drive wheel comprises a plurality of spokes extending from a hub, the spokes being spaced from each other such that a blister cavity locates between successive spokes as the blister strip passes the blister strip drive wheel. The actuating lever engages with the blister strip drive wheel, so that rotation of the actuating lever causes the blister strip drive wheel to rotate and drive the blister strip. The indexing mechanism comprises a drive coupling member which causes the actuating lever and blister strip drive wheel to disengage at the end of the first portion of the forward stroke. Thus the blister strip drive wheel rotates during the first portion of the stroke of the actuating lever so that the next blister is brought into alignment with the piercing element but remains stationary during rotation of the actuating lever through the second portion of its stroke as the piercing element pierces the aligned, and now stationary, blister.

Although piercing of an aligned blister only occurs after movement of the strip has stopped, the indexing mechanism could be configured so that de-coupling of the blister strip drive wheel occurs after the blister piercing element begun to pierce the lid of a blister so that the piercing element is drawn across and through the lid of the blister as it enters it. This creates larger openings than those created when the strip is stationary during piercing, which can help to ensure that the drug dose is entrained in the airflow and removed from the blister.

The indexing mechanism is also preferably configured to inhibit rotation of the blister strip drive wheel when the actuating lever is rotated in the opposite direction during its return stroke.

Preferably the inhaler retains the used blisters. More preferably the inhaler has a wall to separate the interior of the housing into used and unused blister compartments. The wall is preferably rigid and slideably mounted so that the sizes of the unused and used blister compartments change relative to each other as the number of blisters that are used increases and the number of unused blisters decreases. The inhaler may be provided with a flexible and resilient spiral element mounted within the housing into which the used portion of the blister strip is directed so that, as the strip is gradually used up, the spiral expands as more and more of the strip is fed or pushed into it between its coils, as described in WO09/007352 A1.

The used blisters are preferably crushed so that they take up less space. Thus the blister strip drive wheel is preferably positioned such that the distance between the hub and the inner surface of the housing is less than the height of a blister cavity so that onward rotation of the wheel after piercing causes a blister cavity to be at least partially squashed or sandwiched between the hub and the wall.

The cap is preferably pivotally mounted to the housing, so that it can be rotated about a third axis from a closed position in which it covers the mouthpiece to an open position (shown in Figure 1). The cap may be 'passive' in the sense that it can be opened and closed freely without causing indexing of the blister strip or piercing of a blister. However, in a preferred embodiment, the cap and actuating lever include cooperating means configured such that, when the cap is rotated from its open position back into its closed position in which it covers the mouthpiece, the actuating lever is also rotated back into its initial position. Rotation of the actuating lever in turn preferably causes rotation of the mouthpiece back to its original position as the cam drive member travels back along the leg portion of the cam surface.

The cap and actuating lever are preferably configured so that, when the cap is in its closed position and the actuating lever has returned to its initial position, the cap overlies the actuating lever. This prevents a user from attempting to operate the device by rotating the actuating lever prior to opening the cap.

In an alternative embodiment, the cap and the actuating lever are combined into a single component so that rotation of the cap also causes indexing of the strip and piercing of the aligned blister.

The inhaler of the invention may be either passive or active. In a passive inhaler, the dose is entrained in a flow of air caused when the user inhales through the mouthpiece. An active inhaler includes means for generating a pressurised flow of gas or air through the blister to entrain the dose and carry it out of the blister through the mouthpiece and into the user's airway.

The detent mechanism described in WO12/069854 includes a cantilever that extends from the actuating lever and has a kinked region which engages with a pawl that protrudes from the housing. As the actuating lever approaches the end of the second portion of its forward stroke, the cantilever contacts the pawl and is resiliently deflected so that the pawl rides over the kinked region of the cantilever. Once the pawl has cleared the kinked region, the cantilever springs back to its original shape. When the actuating lever is rotated back towards its initial position, the process is reversed so that the cantilever is deflected by the pawl and rides back over it. The biasing force that the detent mechanism applies to the actuating lever depends on the degree of deflection of the cantilever. Thus the force that must be applied to rotate the actuating lever whilst the cantilever is in contact with the pawl is determined by the shape of the kinked region of the cantilever. In fact this shape determines the force that must be applied on both the forward and return strokes of the actuating lever (although the direction of the forces relative to the motion of the cantilever are opposite on the forward and return strokes). So, for example, the kinked region could be designed such that in the forward direction there is a gradual increase in the biasing force until the cantilever reaches its maximum deflection, followed by a sharper decrease to the end of the forward stroke. When returning the actuating lever to the initial position, there is an initial sharp increase in the force required to overcome the hold, followed by a gradual decrease until the cantilever moves out of contact with the pawl. Thus the forward and return force profiles applied by the detent mechanism are the reverse of each other: gentle "uphill" followed by steep "downhill" in the forward direction, and steep "uphill" followed by gentle "downhill" in the reverse direction.

The detent mechanism of WO12/069854 applies the same biasing force at any given point of contact between the cantilever and the pawl, regardless of whether the actuating lever is on the forward or return stroke. A relatively steep downhill slope may be desirable at the end of the forward stroke, in order to pull the actuating lever through the final few degrees of its rotation and to produce an audible click as feedback to the user that end of travel of the actuating lever has been reached. However, this would require an equally steep uphill slope on the return stroke. The optimum steepness for the downhill motion may be too large to allow easy motion on the return and / or could overstress, and hence damage, the cam follower, so a slope which is a compromise must be chosen.

Thus it would be advantageous for the biasing forces applied to the actuator to be different on its forward and return strokes. The present invention solves this problem by providing a mechanism which can apply different biasing forces to the actuating lever on the forward and return strokes, and hence control the force that must be applied to the actuating lever to cause it to rotate

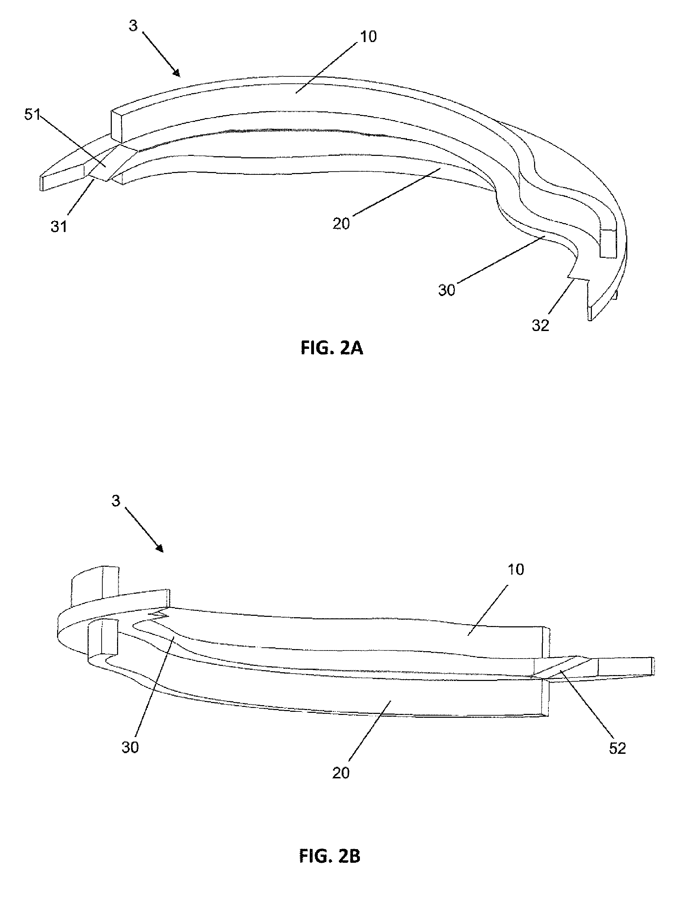

Figure 1a shows a cross-section of an inhaler according to the invention with the actuating lever 1 in its initial position and Figure 1b shows the same view after the actuating lever 1 has been rotated into its piercing position. The mechanism for applying the biasing force to the actuator comprises a cam follower 2 whose head 6 contacts and runs along a track 3 during rotation of the actuating lever 1 from its initial position to its fully depressed position (the forward stroke). The depiction of the track 3 in Figures 1A and 1B is only schematic, and does not show its full structure, which will be described in more detail below with reference to Figures 2, 3 and 4. Whereas the detent mechanism of WO12/069854 only applies a force to the cantilever at the end of the forward stroke and the start of the return stroke, the track allows a biasing force to be applied to the cam follower throughout the whole of, or selected portions of, the forward and return strokes of the actuating lever.

The shape of the track 3 is designed so that the degree of deflection or retraction of the cam follower 2 changes depending on the tactile effect that is required. The ability of the cam follower to deflect or retract may be due to its inherent resilience. Alternatively or additionally, the cam follower may be resiliently mounted on the actuating lever. As the degree of deflection / retraction of the cam follower 2 changes, caused by a change in the slope of the track 3, the force that must be applied to the actuating lever 1 also changes. Thus, the force that must be applied to the actuating lever 1 to overcome the biasing force from the cam follower 2 to cause the actuating lever to rotate can be altered. For example, the track may be designed so that the peak level of force that must be applied to the actuating lever is felt throughout the entire forward stroke, rather than only at a particular point.

In the preferred embodiment described above, the largest force on the actuating lever is typically required when the mouthpiece / piercing element begins to rotate in order to pierce the lid of the aligned blister. Therefore, by shaping the track 3 so that the biasing force applied to the cam follower 2 by the track 3 is relatively high during movement of the actuating lever 1 up until this point, and so that the biasing force is removed or reduced beyond this point, the user can be provided with a constant feel (i.e. the user is required to apply the same force to the actuating lever) throughout the entire stroke of the actuating lever.

The shape of the track can be designed to mask points at which the force applied to the actuating lever changes or to assist travel of the actuating lever, or to provide other advantageous feel or feedback to the user. This may prevent the user from thinking they have reached the end of the stroke of the actuating lever too soon, which could occur for example as a result of feeling a slightly greater resistance to movement at the point at which the mouthpiece or blister piercing element begins to rotate.

As shown in Figure 1, the track 3 may be shaped so that the biasing force that has built up in the cam follower 2 is gradually released at the end of the stroke of the actuating lever. The release of the biasing force on the cam follower has the effect of pulling the actuating lever through its final few degrees of rotation into its final fully actuated position.

A mechanism having a single track would apply the same force to the cam follower regardless of the direction of travel of the actuating lever. However, the mechanism of the present invention applies different biasing forces on the forward and return strokes. This can be achieved by providing a track 3 which has first and second track portions 10, 20 separated by a dividing wall 30, as shown in Figure 2. Figure 2A is a perspective view from above, which mainly shows the first track portion 10 and the dividing wall 30. Figure 2B is a perspective view from below, which mainly shows the second track portion 20 and the dividing wall 30. For reasons that will be explained below, each end 31, 32 of the dividing wall 30 has a ramp 51, 52 respectively.

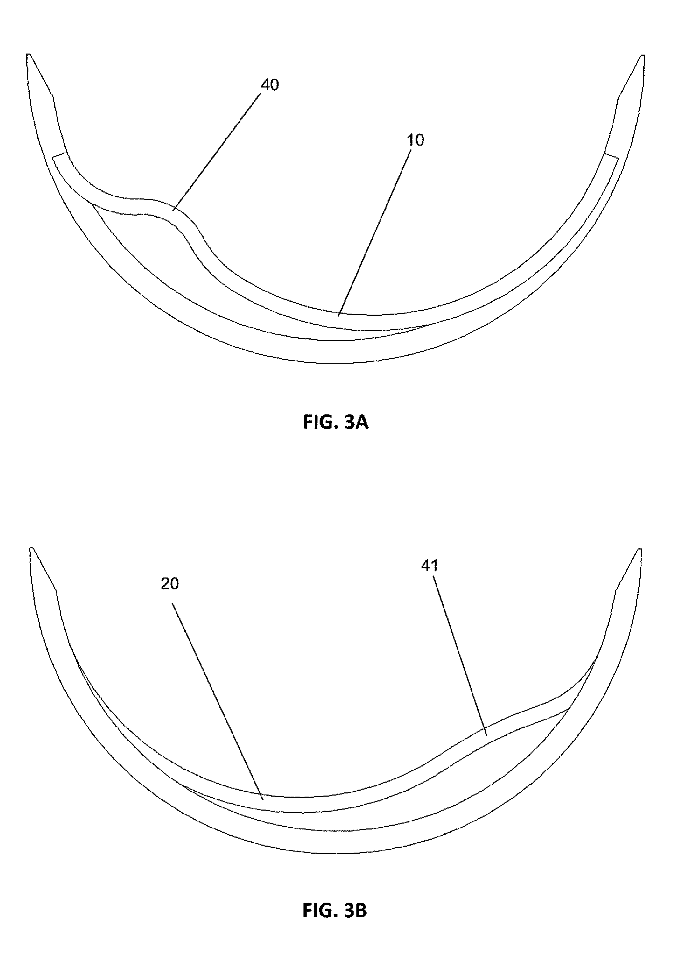

Figures 3A and 3B are schematic diagrams showing the first track portion 10 and the second track portion 20 respectively. The cam follower moves along the first track portion on the forward stroke of the actuating lever (from right to left in Figure 3A) and then takes the second track portion on the return stroke (from left to right in Figure 3B). Since the first and second track portions have different shapes, the biasing forces applied by the cam follower to the actuating lever, and hence the forces that the user applies to cause the actuating lever to rotate, are different on the forward and return strokes. Thus for example, it is possible to have a relatively steep downhill slope at the end of the forward stroke (in order to pull the actuating lever through the final few degrees of its rotation, and to produce an audible click) whilst having a gentle uphill slope which allows easy motion on the return stroke. This results from the hump or protrusion 40 at or near the end (i.e. the left side) of the first track portion of Figure 3A, and the absence of such a hump at the start (i.e. the left side) of the second track portion of Figure 3B respectively. In this example, there is also a smaller hump 41 at or near the end of the second track portion, which produces a similar but smaller effect at the end of the return stroke. It should be noted that either track portion could be omitted completely, thereby allowing the actuating lever to move freely on either the forward or the return stroke.

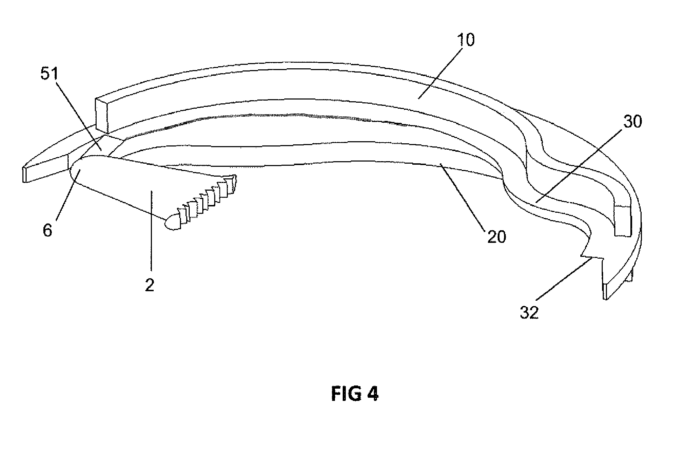

In order to guide the cam follower 2 onto the other track portion at the end of each stroke, each end 31, 32 of the dividing wall 30 has a ramp 51, 52, shown in Figures 2 and 4. When the actuating lever is in its initial position, the head 6 of the cam follower 2 is located just beyond the first end 31 of the dividing wall. The cam follower lies in the plane of the dividing wall so that, if the dividing wall were not present, the cam follower would travel along the location of the dividing wall. As shown in Figure 4, during the initial part of the forward stroke of the actuating lever, the head 6 of the cam follower 2 comes into contact with the ramp 51. The start of the ramp (i.e. the first end 31 of the dividing wall) lies slightly to one side of the central plane of the dividing wall, so the head 6 of the cam follower 2 is guided along the ramp and is deflected or bent to one side by the dividing wall 30. In Figure 4, the first ramp 51 deflects the cam follower 2 upwards, so that the cam follower travels along the first track portion 10 above the dividing wall; however it will be appreciated that the ramp could equally deflect the cam follower downwards so that the first track is on the lower side of the dividing wall.

During further rotation on the forward stroke, the cam follower travels along the first track portion 10, which is shaped to provide the required biasing force profile. When the actuating lever reaches the end of its forward stroke, the cam follower clears the second end 32 of the dividing wall. Since it is no longer deflected or bent by the dividing wall, the head of the cam follower returns to the plane of the dividing wall. On the return stroke of the actuating lever, the ramp 52 guides the head 6 of the cam follower 2 so that it is deflected to the other side of the dividing wall (the lower side in Figure 4) and travels onto the second track portion, which is shaped to produce a different return biasing force profile. When the actuating lever reaches the end of its return stroke (i.e. it comes back to its initial position), the head 6 of the cam follower 2 clears the first end 31 of the dividing wall, so that it returns to the plane of the dividing wall. Thus the cam follower completes a circuit around the dividing wall on each complete cycle of the actuating lever (i.e. one forward and one return stroke). Preferably the head 6 of the cam follower 2 is rounded, as shown in Figure 4, in order to allow smooth motion on the track portions and around the ends of the dividing wall.

Instead of the dividing wall having ramps, a region of the wall adjacent to each end could be angled slightly away from the central plane, so that the head of the cam follower is guided to one side. Alternatively the whole dividing wall could be at a slight angle to the central line of the track so that the dividing wall gradually increases the amount by which the head of the cam follower is deflected or bent throughout the stroke of the actuating lever. These configurations also result in the cam follower making a circuit around the dividing wall.

The cam follower may be configured so that the head bends relative to the body part so that the head is out of the plane of the dividing wall. For example the body part of the cam follower preferably comprises a region of reduced thickness, in particular a line of reduced thickness across the body part, so that when the head portion contacts the dividing wall, the cam follower bends out of the plane of the dividing wall along this line. Alternatively, the cam follower may simply bend as a result of its (at least slightly) resilient nature.

The mechanism of the invention for applying a biasing force to the actuator has a relatively simple construction, is robust, straightforward to manufacture and facilitates use of inhalers by patients.

The disclosures of WO09/007352, WO09/092652 and WO12/069854 as referred to above are all incorporated herein by reference.

Many modifications and variations of the invention falling within the terms of the following claims will be apparent to those skilled in the art and the foregoing description should be regarded as a description of the preferred embodiments of the invention only.

## Claims

1. An inhaler comprising:
• a housing to receive a strip comprising a plurality of blisters having pierceable lids and containing medicament for inhalation;
• a mouthpiece mounted to the housing through which the medicament is inhaled by a user;
• a piercing element;
• an actuator for moving the blisters sequentially into alignment with the piercing element and for subsequently causing the piercing element to pierce the lid of the aligned blister so that when the user inhales through the mouthpiece, an airflow is generated through the blister to entrain the medicament and carry it, via the mouthpiece, into the user's airway; and
• a mechanism for applying a biasing force to the actuator;
**characterised in that** the mechanism is adapted to apply a biasing force to the actuator during a forward stroke which is independent of the biasing force that it applies to the actuator during a return stroke.

2. An inhaler according to claim 1 wherein the mechanism for applying the biasing force comprises a cam follower on the actuator and a track on the housing, wherein the track comprises first and second track portions which are separated by a dividing wall, and wherein the two track portions are differently shaped.

3. An inhaler according to claim 2 wherein the cam follower is resilient.

4. An inhaler according to claim 2 or claim 3 wherein the cam follower is resiliently mounted on the actuating lever.

5. An inhaler according to any of claims 2 to 4 wherein each end of the dividing wall has a ramp to guide the cam follower at the end of each stroke of the actuator from one track portion onto the other track portion for the next stroke.

6. An inhaler according to any of claims 2 to 5 wherein the cam follower has a body part and a head.

7. An inhaler according to claim 6 wherein the head of the cam follower is rounded.

8. An inhaler according to claim 6 or claim 7 wherein the body part of the cam follower comprises a region of reduced thickness so that when the head contacts the dividing wall, the cam follower bends out of the plane of the dividing wall along the region of reduced thickness.

9. An inhaler according to claim 8 wherein the region of reduced thickness is a line of reduced thickness across the body part.

10. An inhaler according to any of claims 2 to 9 wherein the first track portion is shaped such that the biasing force applied to the actuator during the forward stroke gradually increases until the point at which the piercing element begins to pierce the lid of the blister.

11. An inhaler according to claim 10 wherein the biasing force is gradually released after the point at which the piercing element begins to pierce the lid of the blister.

12. An inhaler according to any of claims 2 to 11 wherein the first and / or second track portions are shaped so that near the end of the stroke, the biasing force urges the actuator into a fully actuated and / or fully returned position respectively.

13. An inhaler according to claim 12 wherein the first and / or second track portion comprises a hump or protrusion situated near to the end of the track portion.

14. An inhaler according to any of claims 2 to 13 wherein the cam follower is in contact with the first and / or second track portions throughout a substantial portion of the forward and / or the return strokes of the actuator.

15. An inhaler according to any of claims 1 to 14 wherein the piercing element is mounted for rotation about a first axis and the actuator is an actuating lever mounted for rotation about a second axis, wherein the actuating lever cooperates with the piercing element so that the piercing element pivots about the first axis to pierce the lid of the aligned blister in response to rotation of the actuating lever about the second axis from its initial position.
